(19) Europäisches Patentamt
European Patent Office
Office européen des brevets

(11) EP 1 300 682 A1

(12) EUROPEAN PATENT APPLICATION

(43) Date of publication:
**09.04.2003 Bulletin 2003/15**

(51) Int Cl.[7]: **G01N 33/50**, C12Q 1/48, C07K 14/47

(21) Application number: **02079052.3**

(22) Date of filing: **02.10.2002**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **04.10.2001 US 326973 P**

(71) Applicant: **SMITHKLINE BEECHAM CORPORATION**
**Philadelphia Pennsylvania 19101 (US)**

(72) Inventors:
- **Annan, Roland S.**
  **King of Prussia, Pennsylvania 19406 (US)**
- **Burns, Brian M.**
  **King of Prussia, Pennsylvania 19406 (US)**
- **Creasy, Caretha Lee**
  **King of Prussia, Pennsylvania 19406 (US)**
- **Herold, Kathleen M.**
  **King of Prussia, Pennsylvania 19406 (US)**

(74) Representative: **Thornley, Rachel Mary et al**
**GlaxoSmithKline**
**Corporate Intellectual Property (CN9.25.1)**
**980 Great West Road**
**Brentford, Middlesex TW8 9GS (GB)**

# (54) Methods for finding modulators of DYRK3 and DYRK2

(57) Methods of finding agonists and antagonists to DYRK3 or DYRK2 are provided.

EP 1 300 682 A1

SEQUENCE LISTING

<110> SMITHKLINE BEECHAM CORPORATION
SMITHKLINE BEECHAM p.l.c.

<120> METHODS FOR FINDING MODULATORS OF DYRK3 and DYRK2

<130> GH50044

<140> TO BE ASSIGNED
<141> 2002-09-24

<150> 60/326,973
<151> 2001-10-04

<160> 7

<170> FastSEQ for Windows Version 4.0

<210> 1
<211> 2061
<212> DNA
<213> Homo sapiens

<400> 1
ggagcgaaat gcgctgagct gcagtgtctg gtcgagagta cccgtgggag cgtcgcgccg 60
cggaggcagc cgtcccggcg taggtggcgt ggccgaccgg acccccaact ggcgcctctc 120
cccgcgcggg gtcccgagct aggagatggg aggcacagct cgtgggcctg ggcggaagga 180
tgcggggccg cctggggccg ggctcccgcc ccagcagcgg aggttggggg atggtgtcta 240
tgacaccttc atgatgatag atgaaaccaa atgtcccccc tgttcaaatg tactctgcaa 300
tccttctgaa ccacctccac ccagaagact aaatatgacc actgagcagt ttacaggaga 360
tcatactcag cactttttgg atggaggtga gatgaaggta gaacagctgt ttcaagaatt 420
tggcaacaga aaatccaata ctattcagtc agatggcatc agtgactctg aaaaatgctc 480
tcctactgtt tctcagggta aaagttcaga ttgcttgaat acagtaaaat ccaacagttc 540
atccaaggca cccaaagtgg tgcctctgac tccagaacaa gccctgaagc aatataaaca 600
ccacctcact gcctatgaga aactggaaat aattaattat ccagaaattt actttgtagg 660
tccaaatgcc aagaaaagac atggagttat tggtggtccc aataatggag ggtatgatga 720
tgcagatggg gcctatattc atgtacctcg agaccatcta gcttatcgat atgaggtgct 780
gaaaattatt ggcaagggga gttttgggca ggtggccagg gtctatgatc acaaacttcg 840
acagtacgtg gccctaaaaa tggtgcgcaa tgagaagcgc tttcatcgtc aagcagctga 900
ggagatccgg attttggagc atcttaagaa acaggataaa actggtagta tgaacgttat 960
ccacatgctg gaaagtttca cattccggaa ccatgtttgc atggcctttg aattgctgag 1020
catagacctt tatgagctga ttaaaaaaaa taagtttcag ggttttagcg tccagttggt 1080
acgcaagttt gcccagtcca tcttgcaatc tttggatgcc ctccacaaaa ataagattat 1140
tcactgcgat ctgaagccag aaaacattct cctgaaacac cacgggcgca gttcaaccaa 1200
ggtcattgac tttgggtcca gctgtttcga gtaccagaag ctctacacat atatccagtc 1260
tcggttctac agagctccag aaatcatctt aggaagccgc tacagcacac caattgacat 1320
atggagtttt ggctgcatcc ttgcagaact tttaacagga cagcctctct tccctggaga 1380
ggatgaagga gaccagttgg cctgcatgat ggagcttcta gggatgccac caccaaaact 1440
tctggagcaa tccaaacgtg ccaagtactt tattaattcc aagggcatac cccgctactg 1500
ctctgtgact acccaggcag atgggagggt tgtgcttgtg gggggtcgct cacgtagggg 1560
taaaaagcgg ggtcccccag gcagcaaaga ctgggggaca gcactgaaag ggtgtgatga 1620
ctacttgttt atagagttct tgaaaaggtg tcttcactgg gacccctctg cccgcttgac 1680
cccagctcaa gcattaagac acccttggat tagcaagtct gtccccagac ctctcaccac 1740
catagacaag gtgtcaggga aacgggtagt taatcctgca agtgctttcc agggattggg 1800
ttctaagctg cctccagttg ttggaatagc caataagctt aaagctaact taatgtcaga 1860
aaccaatggt agtatacccc tatgcagtgt attgccaaaa ctgattagct agtggacaga 1920
gatatgccca gagatgcata tgtgtatatt tttatgatct tacaaacctg caaatggaaa 1980
aaatgcaagc ccattggtgg atgtttttgt tagagtagac tttttttaaa caagacaaaa 2040

```
cattttttata tgattataaa a                                              2061

<210> 2
<211> 587
<212> PRT
<213> Homo sapiens

<400> 2
Met Gly Gly Thr Ala Arg Gly Pro Gly Arg Lys Asp Ala Gly Pro Pro
 1               5                  10                  15
Gly Ala Gly Leu Pro Pro Gln Gln Arg Arg Leu Gly Asp Gly Val Asp
            20                  25                  30
Thr Phe Met Met Ile Asp Glu Thr Lys Cys Pro Pro Cys Ser Asn Val
        35                  40                  45
Leu Cys Asn Pro Ser Glu Pro Pro Pro Pro Arg Arg Leu Asn Met Thr
    50                  55                  60
Thr Glu Gln Phe Thr Gly Asp His Thr Gln His Phe Leu Asp Gly Gly
65                  70                  75                  80
Glu Met Lys Val Glu Gln Leu Phe Gln Glu Phe Gly Asn Arg Lys Ser
                85                  90                  95
Asn Thr Ile Gln Ser Asp Gly Ile Ser Asp Ser Glu Lys Cys Ser Pro
            100                 105                 110
Thr Val Ser Gln Gly Lys Ser Ser Asp Cys Leu Asn Thr Val Lys Ser
        115                 120                 125
Asn Ser Ser Ser Lys Ala Pro Lys Val Val Pro Leu Thr Pro Glu Gln
    130                 135                 140
Ala Leu Lys Gln Tyr Lys His His Leu Thr Ala Tyr Glu Lys Leu Glu
145                 150                 155                 160
Ile Ile Asn Tyr Pro Glu Ile Tyr Phe Val Gly Pro Asn Ala Lys Lys
                165                 170                 175
Arg His Gly Val Ile Gly Gly Pro Asn Asn Gly Gly Tyr Asp Asp Ala
            180                 185                 190
Asp Gly Ala Tyr Ile His Val Pro Arg Asp His Leu Ala Tyr Arg Tyr
        195                 200                 205
Glu Val Leu Lys Ile Ile Gly Lys Gly Ser Phe Gly Gln Val Ala Arg
    210                 215                 220
Val Tyr Asp His Lys Leu Arg Gln Tyr Val Ala Leu Lys Met Val Arg
225                 230                 235                 240
Asn Glu Lys Arg Phe His Arg Gln Ala Ala Glu Glu Ile Arg Ile Leu
                245                 250                 255
Glu His Leu Lys Lys Gln Asp Lys Thr Gly Ser Met Asn Val Ile His
            260                 265                 270
Met Leu Glu Ser Phe Thr Phe Arg Asn His Val Cys Met Ala Phe Glu
        275                 280                 285
Leu Leu Ser Ile Asp Leu Tyr Glu Leu Ile Lys Lys Asn Lys Phe Gln
    290                 295                 300
Gly Phe Ser Val Gln Leu Val Arg Lys Phe Ala Gln Ser Ile Leu Gln
305                 310                 315                 320
Ser Leu Asp Ala Leu His Lys Asn Lys Ile Ile His Cys Asp Leu Lys
                325                 330                 335
Pro Glu Asn Ile Leu Leu Lys His His Gly Arg Ser Ser Thr Lys Val
            340                 345                 350
Ile Asp Phe Gly Ser Ser Cys Phe Glu Tyr Gln Lys Leu Tyr Thr Tyr
        355                 360                 365
Ile Gln Ser Arg Phe Tyr Arg Ala Pro Glu Ile Ile Leu Gly Ser Arg
    370                 375                 380
Tyr Ser Thr Pro Ile Asp Ile Trp Ser Phe Gly Cys Ile Leu Ala Glu
385                 390                 395                 400
Leu Leu Thr Gly Gln Pro Leu Phe Pro Gly Glu Asp Glu Gly Asp Gln
                405                 410                 415
Leu Ala Cys Met Met Glu Leu Leu Gly Met Pro Pro Pro Lys Leu Leu
```

```
                420                 425                 430
Glu Gln Ser Lys Arg Ala Lys Tyr Phe Ile Asn Ser Lys Gly Ile Pro
            435                 440                 445
Arg Tyr Cys Ser Val Thr Thr Gln Ala Asp Gly Arg Val Val Leu Val
        450                 455                 460
Gly Gly Arg Ser Arg Arg Gly Lys Lys Arg Gly Pro Pro Gly Ser Lys
465                 470                 475                 480
Asp Trp Gly Thr Ala Leu Lys Gly Cys Asp Asp Tyr Leu Phe Ile Glu
                485                 490                 495
Phe Leu Lys Arg Cys Leu His Trp Asp Pro Ser Ala Arg Leu Thr Pro
            500                 505                 510
Ala Gln Ala Leu Arg His Pro Trp Ile Ser Lys Ser Val Pro Arg Pro
        515                 520                 525
Leu Thr Thr Ile Asp Lys Val Ser Gly Lys Arg Val Val Asn Pro Ala
    530                 535                 540
Ser Ala Phe Gln Gly Leu Gly Ser Lys Leu Pro Pro Val Val Gly Ile
545                 550                 555                 560
Ala Asn Lys Leu Lys Ala Asn Leu Met Ser Glu Thr Asn Gly Ser Ile
                565                 570                 575
Pro Leu Cys Ser Val Leu Pro Lys Leu Ile Ser
            580                 585
```

<210> 3
<211> 551
<212> PRT
<213> Mus musculus

<400> 3

```
Met Met Ile Asp Glu Thr Lys Gly Pro Pro Tyr Ser Asp Thr Phe Ser
 1               5                  10                  15
Asn Pro Ser Glu Ala Pro Val Ser Arg Arg Leu Asn Ile Thr Thr Glu
            20                  25                  30
Pro Leu Thr Arg Gly His Thr Gln His Phe Val Asn Gly Ser Glu Met
        35                  40                  45
Lys Val Glu Gln Leu Phe Gln Glu Phe Gly Asn Arg Arg Ser Asn Thr
    50                  55                  60
Leu Gln Ser Asp Gly Ile Ser Asn Ser Glu Lys Ser Ser Pro Ala Ser
65                  70                  75                  80
Gln Gly Lys Ser Ser Glu Ser Leu Ser Ala Val Lys Cys Asn Leu Ser
                85                  90                  95
Ser Arg Pro Ser Lys Val Leu Pro Leu Thr Pro Glu Gln Ala Leu Lys
            100                 105                 110
Gln Tyr Lys His His Leu Thr Ala Tyr Glu Lys Leu Glu Ile Val Ser
        115                 120                 125
Tyr Pro Glu Ile Tyr Phe Val Gly Pro Asn Ala Lys Lys Arg Gln Gly
    130                 135                 140
Val Ile Gly Gly Pro Asn Asn Gly Gly Tyr Asp Asp Ala Asp Gly Ala
145                 150                 155                 160
Tyr Ile His Val Pro Arg Asp His Leu Ala Tyr Arg Tyr Glu Val Leu
                165                 170                 175
Lys Ile Ile Gly Lys Gly Ser Phe Gly Gln Val Ala Arg Val Tyr Asp
            180                 185                 190
His Lys Leu Arg Gln Tyr Val Ala Leu Lys Met Val Arg Asn Glu Lys
        195                 200                 205
Arg Phe His Arg Gln Ala Ala Glu Glu Ile Arg Ile Leu Glu His Leu
    210                 215                 220
Lys Lys Gln Asp Lys Thr Gly Ser Met Asn Val Ile His Met Leu Glu
225                 230                 235                 240
Ser Phe Thr Phe Arg Asn His Val Cys Met Ala Phe Glu Leu Leu Ser
                245                 250                 255
```

```
Ile Asp Leu Tyr Glu Leu Ile Lys Lys Asn Lys Phe Gln Gly Phe Ser
            260                 265                 270
Val Gln Leu Val Arg Lys Phe Ala Gln Ser Ile Leu Gln Ser Leu Asp
        275                 280                 285
Ala Leu His Lys Asn Lys Ile Ile His Cys Asp Leu Lys Pro Glu Asn
    290                 295                 300
Ile Leu Leu Lys His His Gly Arg Ser Ala Thr Lys Val Ile Asp Phe
305                 310                 315                 320
Gly Ser Ser Cys Phe Glu Tyr Gln Lys Leu Tyr Thr Tyr Ile Gln Ser
                325                 330                 335
Arg Phe Tyr Arg Ala Pro Glu Ile Ile Leu Gly Cys Arg Tyr Ser Thr
            340                 345                 350
Pro Ile Asp Ile Trp Ser Phe Gly Cys Ile Leu Ala Glu Leu Leu Thr
        355                 360                 365
Gly Gln Pro Leu Phe Pro Gly Glu Asp Glu Gly Asp Gln Leu Ala Cys
    370                 375                 380
Met Ile Glu Leu Leu Gly Met Pro Pro Gln Lys Leu Leu Glu Gln Ser
385                 390                 395                 400
Lys Arg Ala Lys Tyr Phe Ile Asn Ser Lys Gly Leu Pro Arg Tyr Cys
                405                 410                 415
Ser Val Ser Thr Gln Thr Asp Gly Arg Val Val Leu Leu Gly Gly Arg
            420                 425                 430
Ser Arg Arg Gly Lys Lys Arg Gly Pro Pro Gly Ser Lys Asp Trp Ala
        435                 440                 445
Thr Ala Leu Lys Gly Cys Gly Asp Tyr Leu Phe Ile Glu Phe Leu Lys
    450                 455                 460
Arg Cys Leu Gln Trp Asp Pro Ser Ala Arg Leu Thr Pro Ala Gln Ala
465                 470                 475                 480
Leu Arg His Pro Trp Ile Ser Lys Ser Thr Pro Lys Pro Leu Thr Met
                485                 490                 495
Asp Lys Val Pro Gly Lys Arg Val Val Asn Pro Thr Asn Ala Phe Gln
            500                 505                 510
Gly Leu Gly Ser Lys Leu Pro Pro Val Val Gly Ile Ala Ser Lys Leu
        515                 520                 525
Lys Ala Asn Leu Met Ser Glu Thr Ser Gly Ser Ile Pro Leu Cys Ser
    530                 535                 540
Val Leu Pro Lys Leu Ile Ser
545                 550
```

<210> 4
<211> 187
<212> PRT
<213> Homo sapiens

<400> 4

```
Met Ser Glu Met Ala Glu Leu Ser Glu Leu Tyr Glu Glu Ser Ser Asp
 1               5                  10                  15
Leu Gln Met Asp Val Met Pro Gly Glu Gly Asp Leu Pro Gln Met Glu
            20                  25                  30
Val Gly Ser Gly Ser Arg Glu Leu Ser Leu Arg Pro Ser Arg Ser Gly
        35                  40                  45
Ala Gln Gln Leu Glu Glu Glu Gly Pro Met Glu Glu Glu Glu Ala Gln
    50                  55                  60
Pro Met Ala Ala Pro Glu Gly Lys Arg Ser Leu Ala Asn Gly Pro Asn
65                  70                  75                  80
Ala Gly Glu Gln Pro Gly Gln Val Ala Gly Ala Asp Phe Glu Ser Glu
                85                  90                  95
Asp Glu Gly Glu Glu Phe Asp Asp Trp Glu Asp Asp Tyr Asp Tyr Pro
            100                 105                 110
Glu Glu Glu Gln Leu Ser Gly Ala Gly Tyr Arg Val Ser Ala Ala Leu
```

```
        115                 120                 125
Glu Glu Ala Asp Lys Met Phe Leu Arg Thr Arg Glu Pro Ala Leu Asp
    130                 135                 140
Gly Gly Phe Gln Met His Tyr Glu Lys Thr Pro Phe Asp Gln Leu Ala
145                 150                 155                 160
Phe Ile Glu Glu Leu Phe Ser Leu Met Val Val Asn Arg Leu Thr Glu
                165                 170                 175
Glu Leu Gly Cys Asp Glu Ile Ile Asp Arg Glu
            180                 185
```

<210> 5
<211> 790
<212> DNA
<213> Homo sapiens

<400> 5

```
gtcgacccac gcgtccgagc ggtttgcaca atgtcggaaa tggctgagtt gtccgagctg 60
tatgaagaga gcagtgacct gcagatggat gtgatgcctg gcgagggtga ccttccgcag 120
atggaggtag gcagcgggag ccgggagcta tccctgcgtc cctcccgcag cggggcccaa 180
cagctcgagg aggaaggccc aatggaggag gaggaggccc agccaatggc ggcgccagag 240
gggaaacgga gccttgctaa cgggcccaac gctggggagc agccaggcca ggtggcgggc 300
gcagacttcg agagcgagga cgagggcgag gaatttgatg actgggagga cgactacgac 360
tatcccgaag aggagcagct cagtggtgcc ggctacagag tatcagccgc tcttgaagaa 420
gccgacaaga tgtttctgag aacaagagaa ccagccctgg atggcgggtt tcagatgcat 480
tatgagaaga ccccgtttga tcagttagct tttatcgaag agcttttttc actgatggtt 540
gtcaatcgtc tgaccgaaga actcggctgt gatgagatta ttgatagaga gtagttagat 600
gctgttaaaa gaggaggaaa ctacttgagg agggacccaa ctttccgcta tcttttgggt 660
tcattccaaa tagttttgtg ccattgaaaa acttgacctt caaaaaaatt tgtttttcag 720
aatagaacac aataggacag tgactgcaca gttgtgaaaa aggaagagaa tcattaaaga 780
aaaaaaaaaa                                                        790
```

<210> 6
<211> 528
<212> PRT
<213> Homo sapiens

<400> 6

```
Met Asn Asp His Leu His Val Gly Ser His Ala His Gly Gln Ile Gln
 1               5                  10                  15
Val Gln Gln Leu Phe Glu Asp Asn Ser Asn Lys Arg Thr Val Leu Thr
            20                  25                  30
Thr Gln Pro Asn Gly Leu Thr Thr Val Gly Lys Thr Gly Leu Pro Val
        35                  40                  45
Val Pro Glu Arg Gln Leu Asp Ser Ile His Arg Arg Gln Gly Ser Ser
    50                  55                  60
Thr Ser Leu Lys Ser Met Glu Gly Met Gly Lys Val Lys Ala Thr Pro
65                  70                  75                  80
Met Thr Pro Glu Gln Ala Met Lys Gln Tyr Met Gln Lys Leu Thr Ala
                85                  90                  95
Phe Glu His His Glu Ile Phe Ser Tyr Pro Glu Ile Tyr Phe Leu Gly
            100                 105                 110
Leu Asn Ala Lys Lys Arg Gln Gly Met Thr Gly Gly Pro Asn Asn Gly
        115                 120                 125
Gly Tyr Asp Asp Asp Gln Gly Ser Tyr Val Gln Val Pro His Asp His
    130                 135                 140
Val Ala Tyr Arg Tyr Glu Val Leu Lys Val Ile Gly Lys Gly Ser Phe
145                 150                 155                 160
Gly Gln Val Val Lys Ala Tyr Asp His Lys Val His Gln His Val Ala
                165                 170                 175
Leu Lys Met Val Arg Asn Glu Lys Arg Phe His Arg Gln Ala Ala Glu
            180                 185                 190
```

```
Glu Ile Arg Ile Leu Glu His Leu Arg Lys Gln Asp Lys Asp Asn Thr
        195                 200                 205
Met Asn Val Ile His Met Leu Glu Asn Phe Thr Phe Arg Asn His Ile
    210                 215                 220
Cys Met Thr Phe Glu Leu Leu Ser Met Asn Leu Tyr Glu Leu Ile Lys
225                 230                 235                 240
Lys Asn Lys Phe Gln Gly Phe Ser Leu Pro Leu Val Arg Lys Phe Ala
                245                 250                 255
His Ser Ile Leu Gln Cys Leu Asp Ala Leu His Lys Asn Arg Ile Ile
            260                 265                 270
His Cys Asp Leu Lys Pro Glu Asn Ile Leu Leu Lys Gln Gln Gly Arg
        275                 280                 285
Ser Gly Ile Lys Val Ile Asp Phe Gly Ser Ser Cys Tyr Glu His Gln
    290                 295                 300
Arg Val Tyr Thr Tyr Ile Gln Ser Arg Phe Tyr Arg Ala Pro Glu Val
305                 310                 315                 320
Ile Leu Gly Ala Arg Tyr Gly Met Pro Ile Asp Met Trp Ser Leu Gly
                325                 330                 335
Cys Ile Leu Ala Glu Leu Leu Thr Gly Tyr Pro Leu Leu Pro Gly Glu
            340                 345                 350
Asp Glu Gly Asp Gln Leu Ala Cys Met Ile Glu Leu Leu Gly Met Pro
        355                 360                 365
Ser Gln Lys Leu Leu Asp Ala Ser Lys Arg Ala Lys Asn Phe Val Ser
    370                 375                 380
Ser Lys Gly Tyr Pro Arg Tyr Cys Thr Val Thr Thr Leu Ser Asp Gly
385                 390                 395                 400
Ser Val Val Leu Asn Gly Gly Arg Ser Arg Arg Gly Lys Leu Arg Gly
                405                 410                 415
Pro Pro Glu Ser Arg Glu Trp Gly Asn Ala Leu Lys Gly Cys Asp Asp
            420                 425                 430
Pro Leu Phe Leu Asp Phe Leu Lys Gln Cys Leu Glu Trp Asp Pro Ala
        435                 440                 445
Val Arg Met Thr Pro Gly Gln Ala Leu Arg His Pro Trp Leu Arg Arg
    450                 455                 460
Arg Leu Pro Lys Pro Pro Thr Gly Glu Lys Thr Ser Val Lys Arg Ile
465                 470                 475                 480
Thr Glu Ser Thr Gly Ala Ile Thr Ser Ile Ser Lys Leu Pro Pro Pro
                485                 490                 495
Ser Ser Ser Ala Ser Lys Leu Arg Thr Asn Leu Ala Gln Met Thr Asp
            500                 505                 510
Ala Asn Gly Asn Ile Gln Gln Arg Thr Val Leu Pro Lys Leu Val Ser
        515                 520                 525


<210> 7
<211> 22
<212> PRT
<213> Homo sapiens

<400> 7
Asp Tyr Pro Glu Glu Glu Gln Leu Ser Gly Ala Gly Tyr Arg Val Ser
 1               5                  10                  15
Ala Ala Leu Glu Glu Ala
            20
```

## Description

### Cross-Reference to Related Applications

This application claims priority to the earlier provisional U.S. application, Serial No. 60/326,973, which was filed on October 4, 2001, the contents of which are herein incorporated by reference in their entirety.

### Field of the Invention

The invention relates to methods of finding agonists and antagonists of human DYRK3, murine DYRK3, and human DYRK2.

### Background

A number of polypeptide growth factors and hormones mediate their cellular effects through a signal transduction pathway. Transduction of signals from the cell surface receptors for these ligands to intracellular effectors frequently involves phosphorylation or dephosphorylation of specific protein substrates by regulatory protein serine/threonine kinases (PSTK) and phosphatases. Serine/threonine phosphorylation is a major mediator of signal transduction in multicellular organisms. Receptor-bound, membrane-bound and intracellular PSTKs regulate cell proliferation, cell differentiation and signaling processes in many cell types. Aberrant protein serine/threonine kinase activity has been implicated or is suspected in a number of pathologies such as rheumatoid arthritis, psoriasis, septic shock, bone loss, many cancers and other proliferative diseases. Accordingly, serine/threonine kinases and the signal transduction pathways which they are part of are potential targets for drug design.

Recently, a mammalian kinase termed DYRK3 was shown to function as a suppressor of red cell development in both human and mouse bone marrow (Lord, *et al*., (2000) *Blood 95,* 2838-2846; Geiger, *et al*., (2001 *Blood* 97, 901-910). Antisense oligonucleotides directed against DYRK3 promote erythroid colony formation of bone marrow cells without affecting other colony forming units. In addition, DYRK3 is expressed predominantly in hematopoietic tissues, and within erythroid or erythropoietin (EPO)-responsive cells in particular. Together, this information suggests that inhibition of DYRK3 could serve as a treatment for anemia.

### Summary of the Invention

In one aspect, the instant invention relates to a method of identifying an agonist or antagonist of human DYRK2 (SEQ ID NO:6), said method comprising the steps of:

(a) incubating a candidate compound with human DYRK2 (SEQ ID NO:6); and
(b) determining the amount of phosphorylation of human EID-1 (SEQ ID NO:4) by human DYRK2 (SEQ ID NO:6); and
(c) identifying a compound that promotes the phosphorylation human EID-1 (SEQ ID NO:4) as an agonist of human DYRK2 (SEQ ID NO:6), and a compound that decreases the phosphorylation of human EID-1 (SEQ ID NO:4) as an antagonist of human DYRK2 (SEQ ID NO:6).

In a second aspect, the instant invention relates to a method of identifying an agonist or antagonist of DYRK3, said method comprising the steps of:

(a) incubating a candidate compound with a DYRK3 selected from group consisting of: murine DYRK2 (SEQ ID NO:3) and human DYRK3 (SEQ ID NO:2); and
(b) measuring the amount of phosphorylation of human EID-1 (SEQ ID NO:4) by said DYRK3; and
(c) identifying a compound that promotes the phosphorylation of human EID-1 (SEQ ID NO:4) as an agonist of said DYRK3 selected from group consisting of: murine DYRK2 (SEQ ID NO:3) and human DYRK3 (SEQ ID NO:2), and a compound that decreases the phosphorylation of human EID-1 (SEQ ID NO:4) as an antagonist of said DYRK3 selected from group consisting of: murine DYRK2 (SEQ ID NO:3) and human DYRK3 (SEQ ID NO:2).

In a third aspect, the instant invention relates to the above-recited method, wherein the DYRK3 is human DYRK3 comprising the amino acid sequence of residues 142-526 of SEQ ID NO:2.

In a fourth aspect, the instant invention relates to any of the above-recited methods, wherein the amount of phosphorylation of human EID-1 (SEQ ID NO:4) is measured using an antibody specific to phosphorylated human EID-1 (SEQ ID NO:4).

In a fifth aspect, the instant invention relates to any of the above-recited methods, wherein the human EID-1 is phosphorylated on serine 125 of human EID-1 (SEQ ID NO:4).

In a sixth aspect, the instant invention relates to any of the above-recited methods, wherein the amount of phosphorylation of human EID-1 (SEQ ID NO:4) is measured by Liquid Chromatography-Electrospray Tandem Mass Spectrometry (LC-ES MS/MS).

In a seventh aspect, the instant invention relates to any of the above-recited methods, wherein the amount of phosphorylation of human EID-1 (SEQ ID NO:4) is measured using a labeled ATP.

In an eighth aspect, the instant invention relates to any of the above-recited methods, wherein the labeled ATP is $P^{33}$ labeled ATP.

## Brief Description of the Drawings

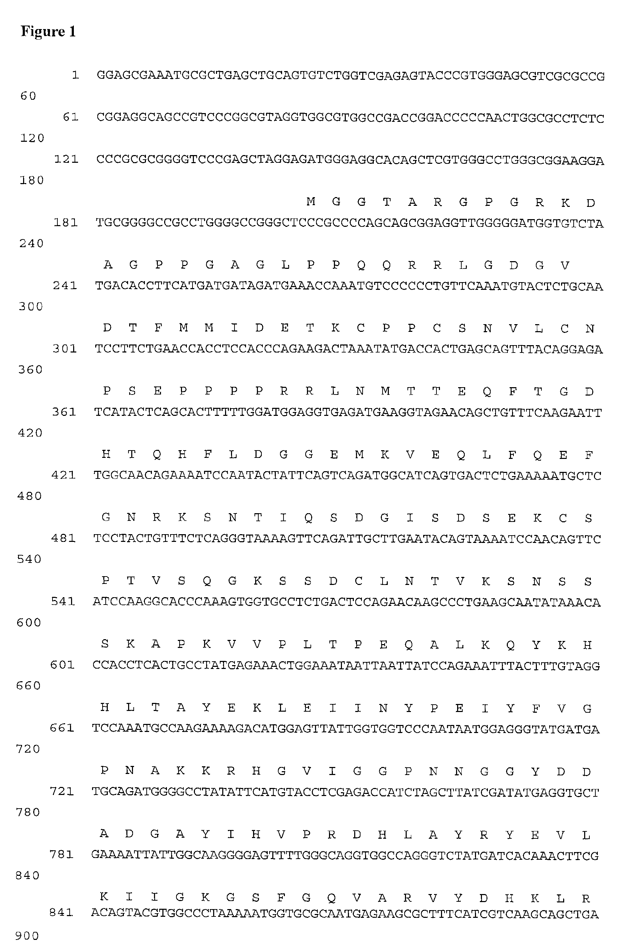

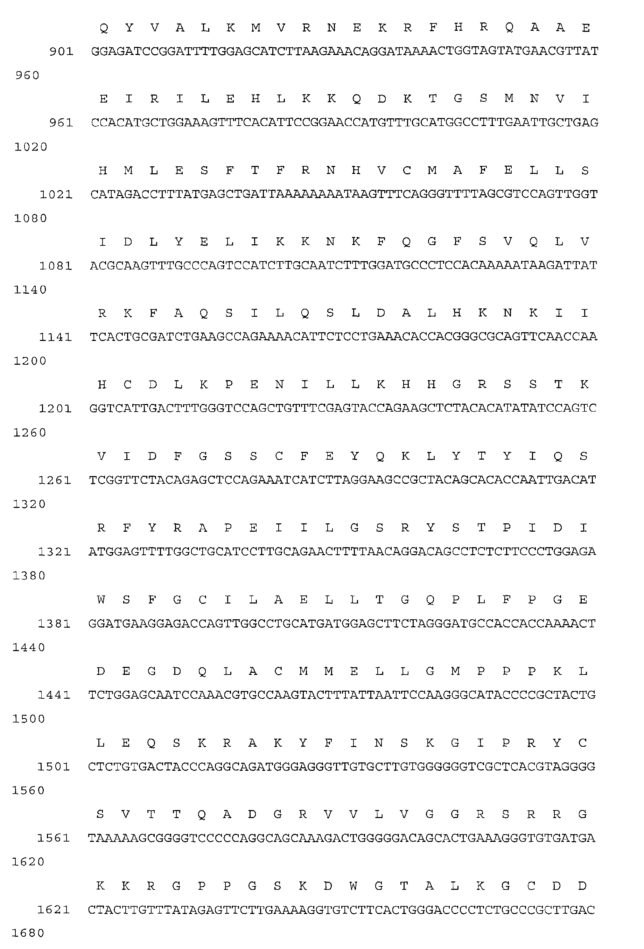

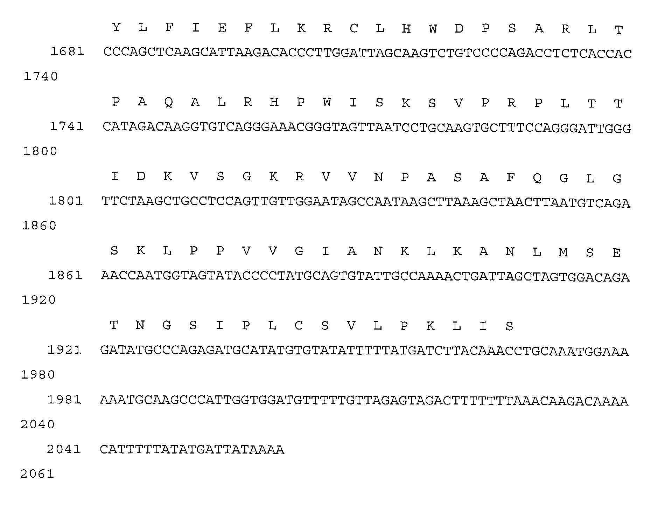

Figure 1 shows the nucleotide and amino acid sequences of human DYRK3 (SEQ NOs:1 and 2, respectively).

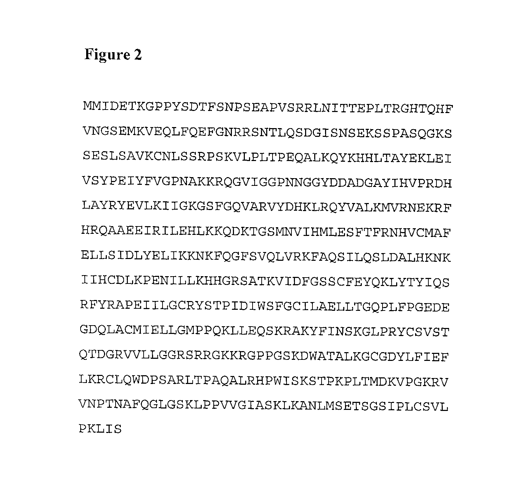

Figure 2 shows the deduced amino acid sequence of murine DYRK3 (SEQ NO:3).

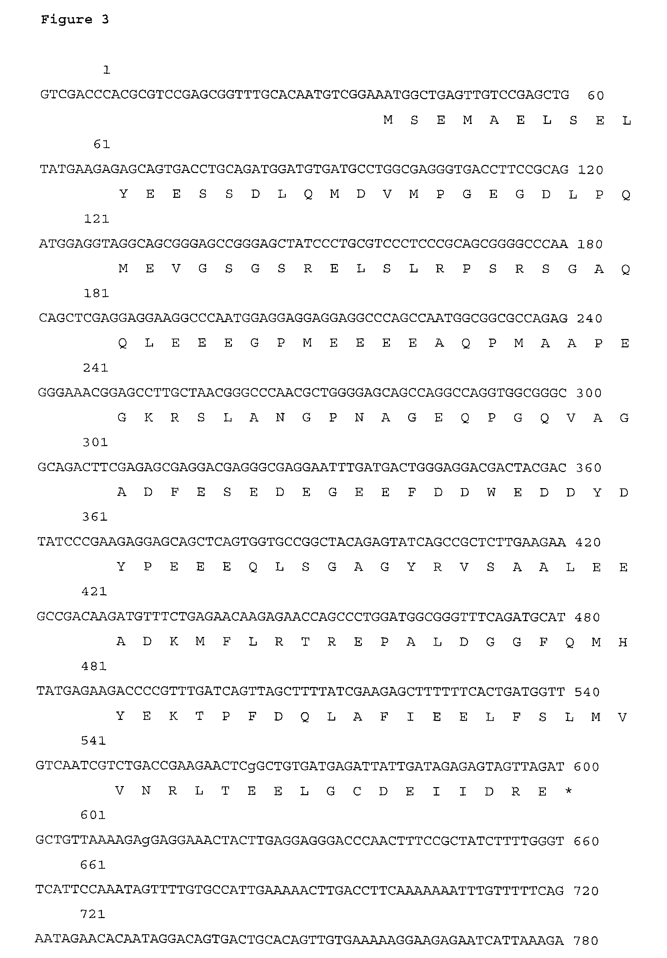

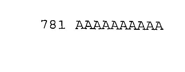

Figure 3 shows the amino acid and nucleotide sequences of human EID-1 (SEQ NOs:4 and 5, respectively).

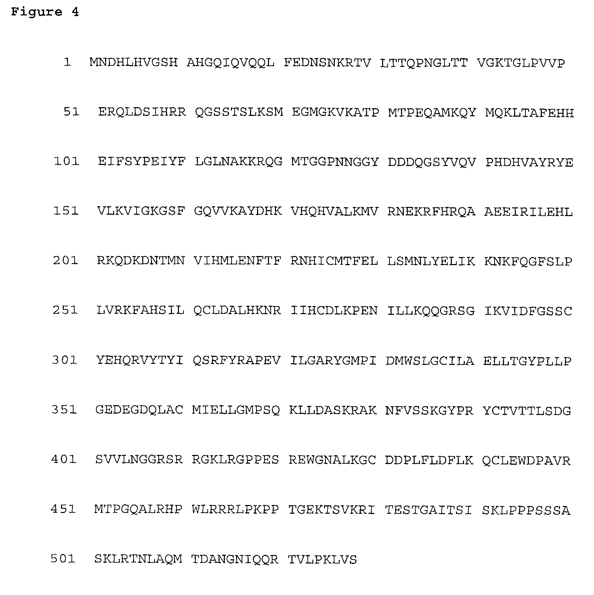

Figure 4 shows the deduced amino acid sequence of human DYRK2 (SEQ ID NO:6).

## Detailed Description of the Invention

Given DYRK2 and DYRK3's potential involvement in erythropoiesis, it is of interest to discover activators and substrates therefor. As disclosed herein, human DYRK3 (hDYRK3) was used as the bait in a yeast two-hybrid screen (Fields, *et al*., (1989) *Nature* **340**, 245-247) to identify human EID-1 as a protein which binds to and is an *in vitro* substrate of DYRK3. Human EID-1 was previously identified as an Rb and p300 -binding protein, and inhibitor of differentiation [Miyake, *et al*., (200) *Mol. Cell. Biol*. **20**, 8889-8902; MacLellan, *et al*., (2000) *Mol. Cell. Biol*. **20**, 8903-8915]. Inhibitors of DYRK2 or DYRK3, in turn, decrease the phosphorylation of human EID-1 (SEQ ID NO:4). Compounds identified that inhibit the phosphorylation of human EID-1 may be useful as therapeutics for erythropoiesis.

The observation discussed below in Example 3 that either DYRK2 or DYRK3 can phosphorylate human EID-1 can be exploited to configure a screening method for identifying a compound which modulates the activity of DYRK3 or DYRK2. More particularly, the method comprises the step of incubating a candidate compound with DYRK3 or DYRK2, and determining the amount of phosphorylation on EID-1 by DYRK3 or DYRK2, whereby a compound which promotes the phosphorylation is an agonist of DYRK3 or DYRK2, and a compound which decreases the phosphorylation is an inhibitor of DYRK3 or DYRK2.

In a more preferred method, DYRK3 is a polypeptide of SEQ ID NO:2 or 3 or a polypeptide with amino acid sequence 142-526 of SEQ ID NO:2, and EID-1 is a polypeptide of SEQ ID NO:4; and DYRK2 is a polypeptide of SEQ ID NO:6. Yet in another preferred method, the amount of phosphorylation is measured using an antibody specific to phosphorylated EID-1 or using a labeled ATP, such as $P^{32}$- or $P^{33}$-ATP.

Using this data generated from the mass spectrometry, discussed below in Example 4, tools such as phosphospecific antibodies directed to a peptide derived from hEID-1 (SEQ ID NO:4) encompassing serine 125, or variants thereof, can be used to identify the phosphorylation state of this residue in cells or tissues expressing hDYRK.3 (SEQ ID NO:2). In addition, one could evaluate the phosphorylation state of hEID-1 (SEQ ID NO:4) in cells or tissues treated with hDYRK3 agonists or inhibitors.

## Definitions

The following definitions are provided to facilitate understanding of certain terms used frequently herein

"DYRK2" (or DYRK2 polypeptides) generally refers to human DYRK2 polypeptide having the amino acid sequence set forth in SEQ ID NO:6 or an allelic variant thereof.
"DYRK3" (or DYRK3 polypeptides) generally refers to human DYRK3 polypeptide having the amino acid sequence set forth in SEQ ID NO:2, or an allelic variant thereof, or to murine DYRK3 having the amino acid sequence set forth in SEQ ID NO:3.
"EID-1" (or EID-1 polypeptides) refers to human EID-1 polypeptide having the amino acid sequence set forth in SEQ ID NO:4 or an allelic variant thereof.

Polypeptides of the present invention also include variants of the aforementioned polypeptides. Such polypeptides vary from the reference polypeptide by insertions, deletions, and substitutions that may be conservative or non-conservative, or any combination thereof. Particularly preferred variants are those in which several, for instance from 50 to 30, from 30 to 20, from 20 to 10, from 10 to 5, from 5 to 3, from 3 to 2, from 2 to 1 or 1 amino acids are inserted, substituted, or deleted, in any combination.

Preferred fragments of polypeptides of the present invention include an isolated polypeptide comprising an amino acid sequence having at least 30, 50 or 100 contiguous amino acids from the amino acid sequence of SEQ ID NOs:2, 3, 4, or 6, or an isolated polypeptide comprising an amino acid sequence having at least 30, 50 or 100 contiguous amino acids truncated or deleted from the amino acid sequence of SEQ ID NOs:2, 3, 4, or 6. Preferred fragments are biologically active fragments that mediate the biological activity of DYRK2, DYRK3, or EID-1, including those with a similar activity or an improved activity, or with a decreased undesirable activity. For example, preferred fragments for DYRK3 include a polypeptide which has the amino acid sequence 142-526 of SEQ ID NO:2, which retains a catalytic activity.

Polypeptides of the present invention can be prepared in any suitable manner, for instance by isolation from naturally occurring sources, from genetically engineered host cells comprising expression systems or by chemical synthesis, using for instance automated peptide synthesizers, or a combination of such methods. Means for preparing such polypeptides are well understood in the art.

"Isolated" means altered "by the hand of man" from its natural state, *i.e.*, if it occurs in nature, it has been changed or removed from its original environment, or both. For example, a polynucleotide or a polypeptide naturally present in a living organism is not "isolated," but the same polynucleotide or polypeptide separated from the coexisting materials of its natural state is "isolated", as the term is employed herein. Moreover, a polynucleotide or polypeptide that is introduced into an organism by transformation, genetic manipulation or by any other recombinant method is "isolated" even if it is still present in said organism, which organism may be living or non-living.

"Polypeptide" refers to any polypeptide comprising two or more amino acids joined to each other by peptide bonds or modified peptide bonds, *i.e.*, peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as post-translational processing, or by chemical modification techniques that are well known in the art. Such modifications are well-described in basic texts and in more detailed monographs, as well as in a voluminous research literature. Modifications may occur anywhere in a polypeptide, including the peptide backbone, the amino acid side-chains and the amino or carboxyl termini. It will be appreciated that the same type of modification may be present to the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from post-translation natural processes or may be made by synthetic methods. Modifications include acetylation, acylation, ADP-ribosylation, amidation, biotinylation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination (see, for instance, Proteins - Structure and Molecular Properties, 2nd Ed., T.E.Creighton, W. H. Freeman and Company, New York, 1993; Wold, F., Post-translational Protein Modifications: Perspectives and Prospects, 1-12, in Post-translational Covalent Modification of Proteins, B. C. Johnson, Ed., Academic Press, New York, 1983; Seifter, *et al*., "Analysis for protein modifications and nonprotein cofactors", *Meth Enzymol*, 182, 626-646, 1990, and Rattan, *et al*., "Protein Synthesis: Post-translational Modifications and Aging", *Ann NY Acad Sci*, 663, 48-62, 1992).

"Fragment" of a polypeptide sequence refers to a polypeptide sequence that is shorter than the reference sequence, and preferably retains essentially the same biological function or activity as the reference polypeptide.

"Variant" refers to a polypeptide that differs from a reference polypeptide, and preferably retains the essential properties thereof. Generally, alterations are limited so that the sequences of the reference polypeptide and the variant are closely similar overall and, in many regions, identical. A variant and reference polypeptide may differ in amino acid sequence by one or more substitutions, insertions, or deletions in any combination. A substituted or inserted amino acid residue may or may not be one encoded by the genetic code. Typical conservative substitutions include Gly, Ala; Val, Ile, Leu; Asp, Glu; Asn, Gln; Ser, Thr; Lys, Arg; and Phe and Tyr. A variant of a polypeptide may be naturally occurring such as an allele, or it may be a variant that is not known to occur naturally. Non-naturally occurring variants of polypeptides may be made by mutagenesis techniques or by direct synthesis. Also included as variants are polypeptides having one or more post-translational modifications, for instance glycosylation, phosphorylation, methylation, ADP ribosylation and the like. Embodiments include methylation of the N-terminal amino acid, phosphorylations of tyrosines, serines, and threonines and modification of C-terminal glycines.

"Identity" reflects a relationship between two or more polypeptide sequences, determined by comparing the sequences. In general, identity refers to an exact amino acid to amino acid correspondence of the two polypeptide sequences, respectively, over the length of the sequences being compared.

"% Identity" - For sequences where there is not an exact correspondence, a "% identity" may be determined. In general, the two sequences to be compared are aligned to give a maximum correlation between the sequences. This may include inserting "gaps" in either one or both sequences, to enhance the degree of alignment. A % identity may be determined over the whole length of each of the sequences being compared (so-called global alignment), that is particularly suitable for sequences of the same or very similar length, or over shorter, defined lengths (so-called local alignment), that is more suitable for sequences of unequal length.

Methods for comparing the identity of two or more sequences are well known in the art. Thus for instance, programs available in the Wisconsin Sequence Analysis Package, version 9.1 (Devereux J, *et al*., *Nucleic Acids Res*, 12, 387-395, 1984, available from Genetics Computer Group, Madison, Wisconsin, USA), BESTFIT and GAP, may be used to determine the % identity between two polypeptide sequences. BESTFIT uses the "local homology" algorithm of Smith and Waterman (*J Mol Biol*, 147,195-197, 1981, Advances in Applied Mathematics, 2, 482-489, 1981) and finds the best single region of similarity between two sequences. BESTFIT is more suited to comparing two polypeptide sequences that are dissimilar in length, the program assuming that the shorter sequence represents a portion of the longer. In comparison, GAP aligns two sequences, finding a "maximum similarity", according to the algorithm of Neddleman and Wunsch (*J Mol Biol*, 48, 443-453, 1970). GAP is more suited to comparing sequences that are approximately the same length and an alignment is expected over the entire length. Preferably, the parameters "Gap Weight" and "Length Weight" used in each program are 12 and 4 for polypeptide sequences, respectively. Preferably, % identities are determined when the two sequences being compared are optimally aligned.

Other programs for determining identity and/or similarity between sequences are also known in the art, for instance the BLAST family of programs (Altschul S.F., *et al., J Mol Biol*, 215, 403-410, 1990, Altschul, S.F., *et al., Nucleic Acids Res*., 25:389-3402, 1997, available from the National Center for Biotechnology Information (NCBI), Bethesda, Maryland, USA and accessible through the home page of the NCBI at www.ncbi.nlm.nih.gov) and FASTA (Pearson W R, *Methods in Enzymology*, 183, 63-99, 1990; Pearson W R and Lipman D J, *Proc Nat Acad Sci USA*, 85, 2444-2448,1988, available as part of the Wisconsin Sequence Analysis Package).

Preferably, the BLOSUM62 amino acid substitution matrix (Henikoff S and Henikoff J G, *Proc. Nat. Acad Sci. USA*, 89, 10915-10919, 1992) is used in polypeptide sequence comparisons including where nucleotide sequences are first translated into amino acid sequences before comparison.

Preferably, the program BESTFIT is used to determine the % identity of a query polypeptide sequence with respect to a reference polypeptide sequence, the query and the reference sequence being optimally aligned and the parameters of the program set at the default value, as hereinbefore described.

"Identity Index" is a measure of sequence relatedness which may be used to compare a candidate sequence (polypeptide) and a reference sequence. Thus, for instance a candidate polypeptide sequence having, for example, an Identity Index of 0.95 compared to a reference polypeptide sequence is identical to the reference sequence except that the polypeptide sequence may include an average of up to five differences per each 100 amino acids of the reference sequence. Such differences are selected from the group consisting of at least one amino acid deletion, substitution, including conservative and non-conservative substitution, or insertion. These differences may occur at the amino- or carboxy-terminal positions of the reference polypeptide sequence or anywhere between these terminal positions, interspersed either individually among the amino acids in the reference sequence or in one or more contiguous groups within the reference sequence. In other words, to obtain a polypeptide sequence having an Identity Index of 0.95 compared to a reference polypeptide sequence, an average of up to 5 in every 100 of the amino acids in the reference sequence may be deleted, substituted or inserted, or any combination thereof, as hereinbefore described. The same applies *mutatis mutandis* for other values of the Identity Index, for instance 0.96, 0.97, 0.98 and 0.99.

The relationship between the number of amino acid differences and the Identity Index may be expressed in the following equation:

$$n_a \leq x_a - (x_a \bullet I),$$

in which:

- $\mathbf{n_a}$ is the number of amino acid differences,
- $\mathbf{x_a}$ is the total number of amino acids in SEQ ID NO:2, 3, or 4,
- **I** is the Identity Index,
- • is the symbol for the multiplication operator, and

in which any non-integer product of $\mathbf{x}_a$ and **l** is rounded down to the nearest integer prior to subtracting it from $\mathbf{x}_a$.

**Biological Methods/Examples**

**Example 1**

Full-length human DYRK3 cDNA (SEQ ID NO: 1) was PCR amplified and cloned into pEG202 (Golemis, *et al*., in *Current Protocols in Molecular Biology*, John Wiley & Sons, New York (1994) using traditional molecular biology methods. The resulting plasmid, pEG202-DYRK3, encoded a fusion protein in which the DNA binding protein, *Lex*A, was fused in-frame to DYRK3. The parent vector, pEG202, was a yeast expression vector which uses the alcohol dehydrogenase (*ADH1*) promoter to express the *Lex*A fusion proteins and *HIS3* as the selectable marker. All procedures, plasmids and strains used in the two-hybrid screen have been described in detail by Golemis, *et al*., *supra*. Yeast strain EGY48B (*MAT*$\alpha$, *trp1, his3, ura3,* 6ops-*LEU2*) was cotransformed with the plasmids pEG202-DYRK3 and pMW107. Transformants were selected using complete minimal media lacking uracil and histidine. The plasmid pPW107 is a yeast expression vector in which eight *Lex*A operator sites are located upstream of a minimal *GAL1* promoter which drives the expression of the *Lac*Z gene, and *URA3* as a selectable marker. Synthesis of the full length LexA-DYRK3 fusion was confirmed by Western blot analysis of yeast extracts using polyclonal antisera directed against LexA. It was confirmed that the LexA-DYRK3 fusion alone was unable to activate the *LEU2* nor *Lac*Z reporter strains.

Strain EGY48 ((*MAT*$\alpha$*, trp1, his3, ura3,* 6ops-*LEU2*) was transformed with a human fetal liver cDNA library in plasmid pJG4-5 using a library scale transformation protocol. This library plasmid contains the *TRP1* selectable marker and allows the expression of cDNAs as fusions (AD fusions) to a cassette containing the SV40 nuclear localization sequence, the acid blob B42, and the hemagglutinin epitope tag. See Gyuris *et al*., *Cell 75,* 791-803 (1993). Expression of this fusion is under control of the galactose inducible promoter *GAL1*. Transformation reactions were plated onto complete minimal media lacking tryptophan. Approximately 4.5 x $10^6$ individual transformants were obtained, pooled and frozen. To ensure that each primary colony containing a library plasmid was mated, 2 x $10^7$ cells (approximately 3 times the number of individual transformants) were mixed with 2 X $10^8$ cells containing pEG202-DYRK3 and pMW107. The mixed cells were incubated on a nitrocellulose/YPD plate overnight at 30 °C to allow mating. Cells were washed off the nitrocellulose and plated onto minimal media lacking uracil, histidine, tryptophan and leucine with galactose/raffinose as the carbon source to induce expression of AD fusions. Colonies arising after 3 and 4 days of growth at 30 °C were picked to complete minimal media lacking uracil, histidine and tryptophan. Colonies containing potential interacting fusion proteins were then tested for galactose dependence and *Lac*Z expression. Those isolates which activated both the *LEU2* and *Lac*Z reporters in a galactose dependent fashion were considered positive and pursued further.

Sequence analysis and subsequent GenBank searches using the BLASTX and BLATSN algorithms with the cDNA sequence or with the deduced amino acid sequence indicated that one of the AD fusion plasmids (isolate YFL11) encoded a protein identical retinoblastoma protein-associated protein (AF109873). Subsequent to this finding Miyake, *et al.* and MacLellan, *et al*. [MCB (2000 20, 8889-8902; MCB (2000) 20, 8903-8915] identified YFL11 as a protein which they term human EID-1 (SEQ ID NO:4).

**Example 2**

To determine whether DYRK3 and EID-1 interact in mammalian cells, murine DYRK3 (SEQ ID NO:3) was engineered for mammalian expression with an N-terminal cmyc6 tag in pCDNA3.1 (Invitrogen) and human EID-1 (SEQ ID NO:4) was engineered with an N-terminal V5-HIS6 tag in p423CDN, using standard molecular techniques. HEK293 cells were transfected with each construct together or singly using FUGENE6 transfection reagent (Roche Molecular Biochemicals). Two days after the transfection, the cells were washed twice in phosphate-buffered saline, and lysed in NP-40 lysis buffer (50 mM Tris-HCl, pH7.5, 150 mM NaCl, 10% glycerol, 1% Nonidet P-40) supplemented with 1X Complete protease inhibitor cocktail (Roche Molecular Biochemicals), 50 mM NaF, 10 mM $\beta$-glycerol phosphate, and 1mM sodium vanadate. Lysates were clarified *via* centrifugation at 12,000 X g for 5 min, and protein concentration determined using BCA protein reagent (Pierce). To immunoprecipitate, 1 ug of anti-V5 antibodies (Invitrogen) and 250 ul of IP buffer [50 mM Tris-HCl, pH7.5, 150 mM NaCl, 10% glycerol, 0.1% Nonidet P-40) supplemented with 1X Complete protease inhibitor cocktail (Roche Molecular Biochemicals)] was added to 300 ug of protein lysate and the reaction mixture incubated at 4 °C overnight. Protein A-Sepharose beads (100 ul of 50% slurry, Life Technologies) were added and incubation continued for 45 min. The beads were washed three times in IP buffer and resuspended in 40 ul 2X SDS sample buffer + 5ul reducing agent (Novex). The material was then separated *via* SDS page, transferred to nitrocellulose and subjected to western blot analysis with anti-cmyc or anti-V5 antibodies using standard conditions. Western blot analysis indicated that V5-EID-1 was immunoprecipitated from HEK293 cells transfected with V5-EID-1 and that cmyc-mDYRK3 is immunoprecipitated only when V5-EID-1 was present. This data, together with the yeast two-hybrid data, indicate that DYRK3 and EID-1 proteins interact.

**Example 3**

To determine if human EID-1 can serve as a substrate for DYRK3 or DYRK2, HEK 293 cells were transfected with p423CDN-V5-EID-1, and V5-EID-1 was immunoprecipitated as described in Example 2. The immunoprecipitated material was then subjected to western blot analysis to confirm the immunoprecipitation and an *in vitro* kinase reaction with purified DYRK3 or DYRK2 protein. To purify DYRK3, the catalytic domain (amino acids 142-526 from SEQ ID NO: 2) was engineered for expression from a Baculovirus expression system with an N-terminal GST-tag and C-terminal HIS6-tag. Following infection of SF9 cells with virus, protein lysate was first passed over a glutathione-sepharose column followed by elution with 10 mM glutathione. Partially purified DYRK3 was then passed over a nickel column and eluted with 250 mM imidazole. To purify DYRK2, the protein with amino acid of SEQ ID NO:6 was engineered for expression from a Baculovirus expression system with an N-terminal HIS6-tag. Following infection of SF9 cells with virus, protein lysate was passed over a nickel column and eluted with 250 mM imidazole. To perform the *in vitro* kinase assay, immunoprecipitated V5-EID-1 or vector alone was incubated with kinase buffer containing 100ng DYRK3 or 50 ng DYRK2 in 25 mM HEPES, pH 7.5, 10 mM $MgCl_2$, 10 mM β-glycerophosphate, 0.2 mM sodium orthovanadate, 50 uM adenosine triphosphate (ATP) and 5 uCi $\gamma$-$^{33}$P-ATP at 37°C for 15 minutes. The reaction was terminated by the addition of sample buffer and boiling followed by electrophoresis and exposure to a Phosphorimager Screen (Molecular Dynamics, Sunnyvale, CA). There is a radioactive band that migrates at the same position as the EID-1 protein in the western blot while this band is absent in the vector alone lane in both the kinase reaction and western blot. This data demonstrates that EID-1 is phosphorylated by DYRK3 and DYRK2 *in vitro.*

In conclusion, the observation that either DYRK2 or DYRK3 can phosphorylate human EID-1 can be exploited to configure a screening method for identifying a compound which modulates the activity of DYRK3 or DYRK2. More particularly, the method comprises the step of incubating a candidate compound with DYRK3 or DYRK2, and determining the amount of phosphorylation on EID-1 by DYRK3 or DYRK2, whereby a compound which promotes the phosphorylation is an agonist of DYRK3 or DYRK2, and a compound which decreases the phosphorylation is an inhibitor of DYRK3 or DYRK2.

In a more preferred method, DYRK3 is a polypeptide of SEQ ID NO:2 or 3 or a polypeptide with amino acid sequence 142-526 of SEQ ID NO:2, and EID-1 is a polypeptide of SEQ ID NO: 4;and DYRK2 is a polypeptide of SEQ ID NO:6. Yet in another preferred method, the amount of phosphorylation is measured using an antibody specific to phosphorylated EID-1 or using a labeled ATP, such as $P^{32}$- or $P^{33}$-ATP.

**Example 4**

Human EID-1 (SEQ ID NO:4) was expressed in *E. coli* as an N-terminal HIS6 fusion and the protein purified by traditional methods known to those skilled in the art. To identify the site(s) on hEID-1 that are phosphorylated by hDYRK3 (SEQ ID NO: 2), an *in vitro* kinase assay was performed as follows: 10 μg purified EID-1 was incubated with kinase buffer containing 600 ng recombinant hDYRK3 in 44 mM HEPES, pH 7.5, 20 mM $MgCl_2$, 7 mM β-glycerophosphate, 0.14 mM sodium orthovanadate, 70 μM EDTA, 0.0018% Tween-20, 9.6 mM β-mercaptoethanol, 50 μM adenosine triphosphate (ATP) with or without 5 μCi g-33P-ATP at room temperature for 1 hr in a 100 μl reaction. The reaction was terminated by the addition of 0.1% TFA followed by electrophoresis and exposure to a Phosphorimager Screen (Molecular Dynamics, Sunnyvale, CA). In the reaction utilizing radioactivity, there was a radioactive band that migrated at the same position as the EID-1 protein following electrophoresis. The corresponding region in the lane that did not contain radioactivity was cut out of the gel and digested with Asp-N followed by Liquid Chromatography-Electrospray Tandem Mass Spectrometry (LC-ES MS/MS) to detect the site of phosphorylation. LC-ES MS/MS is a method that is well understood by those of skill in the art of mass spectrometry. A single phosphorylation site was identified on serine 125 of hEID-1 in the peptide DYPEEEQLSGAGYRVSAALEEA (SEQ ID NO:7). Using this information, tools such as phosphospecific antibodies directed to a peptide derived from hEID-1 (SEQ ID NO:4) encompassing serine 125, or variants thereof, can be used to identify the phosphorylation state of this residue in cells or tissues expressing hDYRK3 (SEQ ID NO:2). In addition, one could evaluate the phosphorylation state of hEID-1 (SEQ ID NO:4) in cells or tissues treated with hDYRK3 agonists or inhibitors.

All publications including, but not limited to, patents and patent applications, cited in this specification, are herein incorporated by reference as if each individual publication were specifically and individually indicated to be incorporated by reference herein as though fully set forth.

The above description fully discloses the invention, including preferred embodiments thereof. Modifications and improvements of the embodiments specifically disclosed herein are within the scope of the following claims. Without further elaboration, it is believed that one skilled in the art can, using the preceding description, utilize the present invention to its fullest extent. Therefore, the examples provided herein are to be construed as merely illustrative and are not a limitation of the scope of the present invention in any way. The embodiments of the invention in which an exclusive property or privilege is claimed are defined as follows.

**Claims**

1. A method of identifying an agonist or antagonist of human DYRK2 (SEQ ID NO:6), said method comprising the steps of:

    (a) incubating a candidate compound with human DYRK2 (SEQ ID NO:6); and
    (b) determining the amount of phosphorylation of human EID-1 (SEQ ID NO:4) by human DYRK2 (SEQ ID NO:6); and
    (c) identifying a compound that promotes the phosphorylation human EID-1 (SEQ ID NO:4) as an agonist of human DYRK2 (SEQ ID NO:6), and a compound that decreases the phosphorylation of human EID-1 (SEQ ID NO:4) as an antagonist of human DYRK2 (SEQ ID NO:6).

2. A method of identifying an agonist or antagonist of DYRK3, said method comprising the steps of:

    (a) incubating a candidate compound with a DYRK3 selected from group consisting of: murine DYRK2 (SEQ ID NO:3) and human DYRK3 (SEQ ID NO:2); and
    (b) measuring the amount of phosphorylation of human EID-1 (SEQ ID NO:4) by said DYRK3; and
    (c) identifying a compound that promotes the phosphorylation of human EID-1 (SEQ ID NO:4) as an agonist of said DYRK3 selected from group consisting of: murine DYRK2 (SEQ ID NO:3) and human DYRK3 (SEQ ID NO:2), and a compound that decreases the phosphorylation of human EID-1 (SEQ ID NO:4) as an antagonist of said DYRK3 selected from group consisting of: murine DYRK2 (SEQ ID NO:3) and human DYRK3 (SEQ ID NO:2).

3. The method as claimed in Claim 2, wherein the DYRK3 is human DYRK3 comprising the amino acid sequence of residues 142-526 of SEQ ID NO:2.

4. The method as claimed in Claim 1, wherein the amount of phosphorylation of human EID-1 (SEQ ID NO:4) is measured using an antibody specific to phosphorylated human EID-1 (SEQ ID NO:4).

5. The method as claimed in Claim 4, wherein the human EID-1 is phosphorylated on serine 125 of human EID-1 (SEQ ID NO:4).

6. The method as claimed in Claim 4, wherein the amount of phosphorylation of human EID-1 (SEQ ID NO:4) is measured by Liquid Chromatography-Electrospray Tandem Mass Spectrometry (LC-ES MS/MS).

7. The method as claimed in Claim 1, wherein the amount of phosphorylation of human EID-1 (SEQ ID NO:4) is measured using a labeled ATP.

8. The method as claimed in Claim 7, wherein the labeled ATP is $P^{33}$ labeled ATP.

9. The method of Claim 2, wherein the amount of phosphorylation of human EID-1 (SEQ ID NO:4) is measured using an antibody specific to phosphorylated human EID-1 (SEQ ID NO:4).

10. The method as claimed in Claim 9, wherein the human EID-1 is phosphorylated on serine 125 of human EID-1 (SEQ ID NO:4).

11. The method as claimed in Claim 9, wherein the amount of phosphorylation of human EID-1 (SEQ ID NO:4) is measured by Liquid Chromatography-Electrospray Tandem Mass Spectrometry (LC-ES MS/MS).

12. The method of Claim 3, wherein the amount of phosphorylation of human EID-1 (SEQ ID NO:4) is measured using an antibody specific to phosphorylated human EID-1 (SEQ ID NO:4).

13. The method as claimed in Claim 12, wherein the human EID-1 is phosphorylated on serine 125 of human EID-1 (SEQ ID NO:4).

14. The method as claimed in Claim 12, wherein the amount of phosphorylation of human EID-1 (SEQ ID NO:4) is measured by Liquid Chromatography-Electrospray Tandem Mass Spectrometry (LC-ES MS/MS).

15. The method as claimed in Claim 3, wherein the amount of phosphorylation of human EID-1 (SEQ ID NO:4) is measured using a labeled ATP.

16. The method as claimed in Claim 15, wherein the labeled ATP is $P^{33}$ labeled ATP.

**Figure 1**

```
     1  GGAGCGAAATGCGCTGAGCTGCAGTGTCTGGTCGAGAGTACCCGTGGGAGCGTCGCGCCG
60
    61  CGGAGGCAGCCGTCCCGGCGTAGGTGGCGTGGCCGACCGGACCCCCAACTGGCGCCTCTC
120
   121  CCCGCGCGGGGTCCCGAGCTAGGAGATGGGAGGCACAGCTCGTGGGCCTGGGCGGAAGGA
180
                                   M  G  G  T  A  R  G  P  G  R  K  D
   181  TGCGGGGCCGCCTGGGGCCGGGCTCCCGCCCCAGCAGCGGAGGTTGGGGGATGGTGTCTA
240
         A  G  P  P  G  A  G  L  P  P  Q  Q  R  R  L  G  D  G  V
   241  TGACACCTTCATGATGATAGATGAAACCAAATGTCCCCCCTGTTCAAATGTACTCTGCAA
300
         D  T  F  M  M  I  D  E  T  K  C  P  P  C  S  N  V  L  C  N
   301  TCCTTCTGAACCACCTCCACCCAGAAGACTAAATATGACCACTGAGCAGTTTACAGGAGA
360
         P  S  E  P  P  P  P  R  R  L  N  M  T  T  E  Q  F  T  G  D
   361  TCATACTCAGCACTTTTTGGATGGAGGTGAGATGAAGGTAGAACAGCTGTTTCAAGAATT
420
         H  T  Q  H  F  L  D  G  G  E  M  K  V  E  Q  L  F  Q  E  F
   421  TGGCAACAGAAAATCCAATACTATTCAGTCAGATGGCATCAGTGACTCTGAAAAATGCTC
480
         G  N  R  K  S  N  T  I  Q  S  D  G  I  S  D  S  E  K  C  S
   481  TCCTACTGTTTCTCAGGGTAAAAGTTCAGATTGCTTGAATACAGTAAAATCCAACAGTTC
540
         P  T  V  S  Q  G  K  S  S  D  C  L  N  T  V  K  S  N  S  S
   541  ATCCAAGGCACCCAAAGTGGTGCCTCTGACTCCAGAACAAGCCCTGAAGCAATATAAACA
600
         S  K  A  P  K  V  V  P  L  T  P  E  Q  A  L  K  Q  Y  K  H
   601  CCACCTCACTGCCTATGAGAAACTGGAAATAATTAATTATCCAGAAATTTACTTTGTAGG
660
         H  L  T  A  Y  E  K  L  E  I  I  N  Y  P  E  I  Y  F  V  G
   661  TCCAAATGCCAAGAAAAGACATGGAGTTATTGGTGGTCCCAATAATGGAGGGTATGATGA
720
         P  N  A  K  K  R  H  G  V  I  G  G  P  N  N  G  G  Y  D  D
   721  TGCAGATGGGGCCTATATTCATGTACCTCGAGACCATCTAGCTTATCGATATGAGGTGCT
780
         A  D  G  A  Y  I  H  V  P  R  D  H  L  A  Y  R  Y  E  V  L
   781  GAAAATTATTGGCAAGGGGAGTTTTGGGCAGGTGGCCAGGGTCTATGATCACAAACTTCG
840
         K  I  I  G  K  G  S  F  G  Q  V  A  R  V  Y  D  H  K  L  R
   841  ACAGTACGTGGCCCTAAAAATGGTGCGCAATGAGAAGCGCTTTCATCGTCAAGCAGCTGA
900
```

Q Y V A L K M V R N E K R F H R Q A A E

901 GGAGATCCGGATTTTGGAGCATCTTAAGAAACAGGATAAAACTGGTAGTATGAACGTTAT

960

E I R I L E H L K K Q D K T G S M N V I

961 CCACATGCTGGAAAGTTTCACATTCCGGAACCATGTTTGCATGGCCTTTGAATTGCTGAG

1020

H M L E S F T F R N H V C M A F E L L S

1021 CATAGACCTTTATGAGCTGATTAAAAAAAATAAGTTTCAGGGTTTTAGCGTCCAGTTGGT

1080

I D L Y E L I K K N K F Q G F S V Q L V

1081 ACGCAAGTTTGCCCAGTCCATCTTGCAATCTTTGGATGCCCTCCACAAAAATAAGATTAT

1140

R K F A Q S I L Q S L D A L H K N K I I

1141 TCACTGCGATCTGAAGCCAGAAAACATTCTCCTGAAACACCACGGGCGCAGTTCAACCAA

1200

H C D L K P E N I L L K H H G R S S T K

1201 GGTCATTGACTTTGGGTCCAGCTGTTTCGAGTACCAGAAGCTCTACACATATATCCAGTC

1260

V I D F G S S C F E Y Q K L Y T Y I Q S

1261 TCGGTTCTACAGAGCTCCAGAAATCATCTTAGGAAGCCGCTACAGCACACCAATTGACAT

1320

R F Y R A P E I I L G S R Y S T P I D I

1321 ATGGAGTTTTGGCTGCATCCTTGCAGAACTTTTAACAGGACAGCCTCTCTTCCCTGGAGA

1380

W S F G C I L A E L L T G Q P L F P G E

1381 GGATGAAGGAGACCAGTTGGCCTGCATGATGGAGCTTCTAGGGATGCCACCACCAAAACT

1440

D E G D Q L A C M M E L L G M P P P K L

1441 TCTGGAGCAATCCAAACGTGCCAAGTACTTTATTAATTCCAAGGGCATACCCCGCTACTG

1500

L E Q S K R A K Y F I N S K G I P R Y C

1501 CTCTGTGACTACCCAGGCAGATGGGAGGGTTGTGCTTGTGGGGGGTCGCTCACGTAGGGG

1560

S V T T Q A D G R V V L V G G R S R R G

1561 TAAAAAGCGGGGTCCCCCAGGCAGCAAAGACTGGGGGACAGCACTGAAAGGGTGTGATGA

1620

K K R G P P G S K D W G T A L K G C D D

1621 CTACTTGTTTATAGAGTTCTTGAAAAGGTGTCTTCACTGGGACCCCTCTGCCCGCTTGAC

1680

Y L F I E F L K R C L H W D P S A R L T

1681 CCCAGCTCAAGCATTAAGACACCCTTGGATTAGCAAGTCTGTCCCCAGACCTCTCACCAC 1740

P A Q A L R H P W I S K S V P R P L T T

1741 CATAGACAAGGTGTCAGGGAAACGGGTAGTTAATCCTGCAAGTGCTTTCCAGGGATTGGG 1800

I D K V S G K R V V N P A S A F Q G L G

1801 TTCTAAGCTGCCTCCAGTTGTTGGAATAGCCAATAAGCTTAAAGCTAACTTAATGTCAGA 1860

S K L P P V V G I A N K L K A N L M S E

1861 AACCAATGGTAGTATACCCCTATGCAGTGTATTGCCAAAACTGATTAGCTAGTGGACAGA 1920

T N G S I P L C S V L P K L I S

1921 GATATGCCCAGAGATGCATATGTGTATATTTTTATGATCTTACAAACCTGCAAATGGAAA 1980

1981 AAATGCAAGCCCATTGGTGGATGTTTTTGTTAGAGTAGACTTTTTTTAAACAAGACAAAA 2040

2041 CATTTTTATATGATTATAAAA 2061

**Figure 2**

MMIDETKGPPYSDTFSNPSEAPVSRRLNITTEPLTRGHTQHF
VNGSEMKVEQLFQEFGNRRSNTLQSDGISNSEKSSPASQGKS
SESLSAVKCNLSSRPSKVLPLTPEQALKQYKHHLTAYEKLEI
VSYPEIYFVGPNAKKRQGVIGGPNNGGYDDADGAYIHVPRDH
LAYRYEVLKIIGKGSFGQVARVYDHKLRQYVALKMVRNEKRF
HRQAAEEIRILEHLKKQDKTGSMNVIHMLESFTFRNHVCMAF
ELLSIDLYELIKKNKFQGFSVQLVRKFAQSILQSLDALHKNK
IIHCDLKPENILLKHHGRSATKVIDFGSSCFEYQKLYTYIQS
RFYRAPEIILGCRYSTPIDIWSFGCILAELLTGQPLFPGEDE
GDQLACMIELLGMPPQKLLEQSKRAKYFINSKGLPRYCSVST
QTDGRVVLLGGRSRRGKKRGPPGSKDWATALKGCGDYLFIEF
LKRCLQWDPSARLTPAQALRHPWISKSTPKPLTMDKVPGKRV
VNPTNAFQGLGSKLPPVVGIASKLKANLMSETSGSIPLCSVL
PKLIS

Figure 3

```
       1
GTCGACCCACGCGTCCGAGCGGTTTGCACAATGTCGGAAATGGCTGAGTTGTCCGAGCTG  60
                                            M  S  E  M  A  E  L  S  E  L
      61
TATGAAGAGAGCAGTGACCTGCAGATGGATGTGATGCCTGGCGAGGGTGACCTTCCGCAG 120
       Y  E  E  S  S  D  L  Q  M  D  V  M  P  G  E  G  D  L  P  Q
     121
ATGGAGGTAGGCAGCGGGAGCCGGGAGCTATCCCTGCGTCCCTCCCGCAGCGGGGCCCAA 180
       M  E  V  G  S  G  S  R  E  L  S  L  R  P  S  R  S  G  A  Q
     181
CAGCTCGAGGAGGAAGGCCCAATGGAGGAGGAGGAGGCCCAGCCAATGGCGGCGCCAGAG 240
       Q  L  E  E  E  G  P  M  E  E  E  E  A  Q  P  M  A  A  P  E
     241
GGGAAACGGAGCCTTGCTAACGGGCCCAACGCTGGGGAGCAGCCAGGCCAGGTGGCGGGC 300
       G  K  R  S  L  A  N  G  P  N  A  G  E  Q  P  G  Q  V  A  G
     301
GCAGACTTCGAGAGCGAGGACGAGGGCGAGGAATTTGATGACTGGGAGGACGACTACGAC 360
       A  D  F  E  S  E  D  E  G  E  E  F  D  D  W  E  D  D  Y  D
     361
TATCCCGAAGAGGAGCAGCTCAGTGGTGCCGGCTACAGAGTATCAGCCGCTCTTGAAGAA 420
       Y  P  E  E  E  Q  L  S  G  A  G  Y  R  V  S  A  A  L  E  E
     421
GCCGACAAGATGTTTCTGAGAACAAGAGAACCAGCCCTGGATGGCGGGTTTCAGATGCAT 480
       A  D  K  M  F  L  R  T  R  E  P  A  L  D  G  G  F  Q  M  H
     481
TATGAGAAGACCCCGTTTGATCAGTTAGCTTTTATCGAAGAGCTTTTTTCACTGATGGTT 540
       Y  E  K  T  P  F  D  Q  L  A  F  I  E  E  L  F  S  L  M  V
     541
GTCAATCGTCTGACCGAAGAACTCgGCTGTGATGAGATTATTGATAGAGAGTAGTTAGAT 600
       V  N  R  L  T  E  E  L  G  C  D  E  I  I  D  R  E  *
     601
GCTGTTAAAAGAgGAGGAAACTACTTGAGGAGGGACCCAACTTTCCGCTATCTTTTGGGT 660
     661
TCATTCCAAATAGTTTTGTGCCATTGAAAAACTTGACCTTCAAAAAAATTTGTTTTTCAG 720
     721
AATAGAACACAATAGGACAGTGACTGCACAGTTGTGAAAAAGGAAGAGAATCATTAAAGA 780
```

781 AAAAAAAAAA

Figure 4

```
1   MNDHLHVGSH AHGQIQVQQL FEDNSNKRTV LTTQPNGLTT VGKTGLPVVP

51  ERQLDSIHRR QGSSTSLKSM EGMGKVKATP MTPEQAMKQY MQKLTAFEHH

101 EIFSYPEIYF LGLNAKKRQG MTGGPNNGGY DDDQGSYVQV PHDHVAYRYE

151 VLKVIGKGSF GQVVKAYDHK VHQHVALKMV RNEKRFHRQA AEEIRILEHL

201 RKQDKDNTMN VIHMLENFTF RNHICMTFEL LSMNLYELIK KNKFQGFSLP

251 LVRKFAHSIL QCLDALHKNR IIHCDLKPEN ILLKQQGRSG IKVIDFGSSC

301 YEHQRVYTYI QSRFYRAPEV ILGARYGMPI DMWSLGCILA ELLTGYPLLP

351 GEDEGDQLAC MIELLGMPSQ KLLDASKRAK NFVSSKGYPR YCTVTTLSDG

401 SVVLNGGRSR RGKLRGPPES REWGNALKGC DDPLFLDFLK QCLEWDPAVR

451 MTPGQALRHP WLRRRLPKPP TGEKTSVKRI TESTGAITSI SKLPPPSSSA

501 SKLRTNLAQM TDANGNIQQR TVLPKLVS
```

European Patent Office

# EUROPEAN SEARCH REPORT

Application Number
EP 02 07 9052

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | EP 0 870 825 A (SMITHKLINE BEECHAM CORP) 14 October 1998 (1998-10-14) --- | | G01N33/50<br>C12Q1/48<br>C07K14/47 |
| A | CAMPBELL L E ET AL: "Differing substrate specificities of members of the DYRK family of arginine-directed protein kinases" FEBS LETTERS, ELSEVIER SCIENCE PUBLISHERS, AMSTERDAM, NL, vol. 510, no. 1-2, 2 January 2002 (2002-01-02), pages 31-36, XP004330500 ISSN: 0014-5793 --- | | |
| A | MIYAKE S ET AL: "CELLS DEGRADE A NOVEL INHIBITOR OF DIFFERENTIATION WITH E1A-LIKE PROPERTIES UPON EXITING THE CELL CYCLE" MOLECULAR AND CELLULAR BIOLOGY, AMERICAN SOCIETY FOR MICROBIOLOGY, WASHINGTON, US, vol. 20, no. 23, December 2000 (2000-12), pages 8889-8902, XP002953868 ISSN: 0270-7306 ----- | | TECHNICAL FIELDS SEARCHED (Int.Cl.7)<br>G01N |

The present search report has been drawn up for all claims

| Place of search | Date of completion of the search | Examiner |
|---|---|---|
| MUNICH | 30 January 2003 | Stricker, J-E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 02 07 9052

This annex lists the patent family members relating to the patent documents cited in the above-mentioned European search report.
The members are as contained in the European Patent Office EDP file on
The European Patent Office is in no way liable for these particulars which are merely given for the purpose of information.

30-01-2003

| Patent document cited in search report | Publication date | Patent family member(s) | Publication date |
|---|---|---|---|
| EP 0870825 A | 14-10-1998 | US 5965420 A | 12-10-1999 |
| | | CA 2231046 A1 | 05-09-1998 |
| | | DE 69802131 D1 | 29-11-2001 |
| | | EP 0870825 A1 | 14-10-1998 |
| | | JP 11000179 A | 06-01-1999 |
| | | US 6165766 A | 26-12-2000 |

EPO FORM P0459

For more details about this annex : see Official Journal of the European Patent Office, No. 12/82